# EUROPÄISCHE PATENTSCHRIFT

(11) **EP 1 189 661 B1**
(45) Veröffentlichungstag und Bekanntmachung des Hinweises auf die Patenterteilung: **28.11.2012**
(21) Anmeldenummer: 00934838.4
(22) Anmeldetag: 20.06.2000
(51) Int. Cl.: A61N 5/10

(54) **VORRICHTUNG ZUM DURCHFÜHREN EINER PROTONENTHERAPIE**
DEVICE FOR CARRYING OUT PROTON THERAPY
DISPOSITIF POUR LA MISE EN OEUVRE D'UNE THERAPIE PROTONIQUE

(30) Priorität: 25.06.1999 CH 118099
(43) Veröffentlichungstag der Anmeldung: 27.03.2002
(73) Patentinhaber: PAUL SCHERRER INSTITUT, 5232 Villigen PSI (CH)
(72) Erfinder: PEDRONI, Eros, CH-5200 Brugg (CH)
(74) Vertreter: Fischer, Michael
(86) Internationale Anmeldenummer: PCT/CH2000/000334
(87) Internationale Veröffentlichungsnummer: WO 2001/000276

(56) Entgegenhaltungen:
- EP-A- 0 173 926
- EP-A- 0 864 337
- EP-A- 0 911 064
- FR-A- 2 702 663
- US-A- 5 260 581
- PEDRONI E ET AL: "THE 200-MEV PROTON THERAPY PROJECT AT THE PAUL SCHERRER INSTITUTE: CONCEPTUAL DESIGN AND PRACTICAL REALIZATION" MEDICAL PHYSICS,US,AMERICAN INSTITUTE OF PHYSICS. NEW YORK, Bd. 22, Nr. 1, 1995, Seiten 37-53, XP000505145 ISSN: 0094-2405 in der Anmeldung erwähnt

## Beschreibung

Die vorliegende Erfindung betrifft eine Vorrichtung zur Durchführung einer Protonentherapie an Menschen sowie verschiedene Verbesserungen zur Erhöhung der Sicherheit, zur Verbesserung und Vereinfachung der Prozessführung, zur Erhöhung der Patientenfreundlichkeit sowie um eine kleinere Dimensionierung der Anlage zu ermöglichen sowie eine Verwendung oder Vorrichtung.

Die Protonentherapie, insbesondere für die Behandlung von Krebserkrankungen, gewinnt mehr und mehr an Bedeutung, da sie gegenüber der weit verbreiteten Photonen-Bestrahlungstherapie gewichtige Vorteile mit sich bringt.

Anlagen zur Durchführung der Protonentherapie sind wohl seit Mitte der Fünfziger Jahre in den USA bekannt, trotzdem werden bis heute derartige Therapien nur an wenigen Zentren, wie insbesondere an Forschungsanstalten, weltweit durchgeführt. Dies einerseits aufgrund der nach wie vor teuren Protonenbeschleunigungsanlagen, welche notwendig sind, und andererseits auch aufgrund der voluminösen und komplizierten Protonentherapieanlagen, welche für die Durchführung einer effizienten und sicheren Therapie notwendig sind. Die erste und einzige rein spitalbasierende Protonentherapieanlage steht im Loma Linda University Medical Center in Kalifornien, USA. Weitere Anlagen sind in der Phase der Inbetriebnahme in Boston (USA) und Kashiwa (Japan).

Im Gegensatz zu der erwähnten Anlage am Loma Linda University Medical Center, wo die Protonentherapie mittels einer sogenannten scattering-Technik durchgeführt wird, wurde am Paul Scherrer Institut in Würenlingen, Schweiz, eine Protonentherapieanlage entwickelt, welche die sogenannte spot-scanning-Technik verwendet. In diesem Zusammenhang sei verwiesen auf einen Artikel von Eros Pedroni et al., Med.Phys. 22 (1), Januar 1995, Seiten 37 - 53, mit dem Titel "The 200-MeV proton therapy project at the Paul Scherrer Institute: Conceptual design and practical realization." In diesem Artikel wird auf das grundsätzliche Prinzip der erwähnten spot-scanning-Technik hingewiesen sowie eine Anlage beschrieben mit der Bezeichnung "gantry", mittels welcher Anlage seit ca. 3 Jahren Protonentherapien an Patienten durchgeführt werden, für die Behandlung von Krebserkrankungen. Obwohl die Anlage am Paul Scherrer Institut durch die Verwendung der sogenannten spot-scanning-Technik gegenüber der Anlage am Loma Linda University Medical Center in ihren äusseren Dimensionen reduziert werden konnte, weist diese Anlage nach wie vor einen Durchmesser von ca. 4 m auf, und zudem ist die Zugänglichkeit zum Patienten während der Behandlung unbefriedigend. Auf eine detaillierte Beschreibung der Anlage am Paul Scherrer Institut kann unter Hinweis der obenerwähnten Literatur verzichtet werden, indem diese Literaturstelle integraler Bestandteil der vorliegenden Patentanmeldung ist.

In den europäischen Patentanmeldungen EP 0 864 337 und EP 0 911 064 werden ähnliche Anordnungen zum Behandeln eines Patienten mittels Protonentherapie beschrieben, welche teilweise auf der im Paul Scherrer Institut entwickelten Anlage basieren bzw. ähnliche oder gleiche Behandlungsverfahren beschreiben.

Die bevorzugte Position eines Patienten ist liegend, um eine Deformation der Organe bei der Behandlung zu vermeiden. Somit muss eine allseitig zugängliche und den ganzen Bereich eines menschlichen Körpers umfassende Therapie möglich sein, weshalb in der Regel bekannte Protonentherapieanlagen, wie auch diejenige beim Paul Scherrer Institut, so ausgelegt sind, dass das ganze Protonenstrahlführungsgehäuse um 360° um den sogenannten Patiententisch herum um eine Zentralachse drehbar ist, womit die Anlage einen Durchmesser von 4 bis 12 Metern messen kann. Insbesondere bei einer Behandlung eines Patienten von unten muss die Protonenstrahlführung unter den Patiententisch geführt werden resp. wird der Patiententisch auf eine Position angehoben, welche um einige Meter über dem eigentlichen Arbeitsbodenniveau zu liegen kommt. Die damit verbundenen Nachteile können insbesondere ebenfalls der obenerwähnten Literaturstelle auf Seite 49 unter Kapitel IV, D4, entnommen werden, wo auf die Problematik dieses Anhebens des Patiententisches hingewiesen wird. Diese Positionierung ist kritisch und bedarf im Falle einer Panne der Anlage während der Behandlung einer speziellen Kraneinrichtung, um den Patienten bergen bzw. betreuen zu können. Wohl kann dieser Nachteil insofern gemindert werden, indem im Boden unter dem Patiententisch ein relativ tiefer Schacht angeordnet wird, doch ergibt sich damit die Gefahr von Unfällen, indem beispielsweise die einen Patienten betreuende Person in diesen Schacht abstürzen kann.

Es ist daher eine Aufgabe der vorliegenden Erfindung, Massnahmen vorzuschlagen, mittels welchen eine Protonentherapie in ihrem Betrieb vereinfacht und sicherer gemacht werden kann und vorzugsweise in ihren äusseren Dimensionen reduziert werden kann.

Erfindungsgemäss wird die gestellte Aufgabe mittels einer Protonentherapieanlage gemäss dem Wortlaut nach Anspruch 1 gelöst.

Erfindungsgemäss wird vorgeschlagen, dass eine Protonenstrahlführungs- und -steuerungseinrichtung bzw. ein in der Behandlungsanordnung angeordnetes Protonenstrahlführungsgehäuse entgegen dem in der Literatur beschriebenen "gantry" im Paul Scherer Institut nicht um volle 360° um einen Patiententisch herum rotiert werden kann, sondern dass die Rotationsbewegung auf ca. 270° beschränkt wird. Dabei erfolgt die Rotation im wesentlichen um eine horizontale Drehachse, in welcher Drehachse in der Regel in Ausgangsposition ein steuerbar bewegbarer Patiententisch angeordnet ist. Durch diese Begrenzung auf 270° ergibt sich somit ein Bereich, durch welchen hindurch die Strahlführungs- und -steuerungseinrichtung nicht bewegbar ist, in welchem Bereich einerseits der Patiententisch frei bewegbar ist und zudem der Patiententisch jederzeit frei zugänglich ist. Speziell diese Zugänglichkeit zum Patiententisch stellt eine wesentliche Verbesserung der vorliegenden Erfindung dar, indem jederzeit Betreuungspersonen gefahrlos und unbehindert zum Patienten gelangen können. Durch die Anordnung der Protonenstrahlführungs- und -Steuereinrichtung derart, dass sie aus der Horizontalebene, welche in etwa durch die Drehachse verläuft, sowohl aufwärts wie nach unten um ca. 135° um die Drehachse bzw. von -90° bis +180° von der Vertikalen rotierbar ist, ist somit der Patiententisch von der gegenüberliegenden Seite frei zugänglich. Somit ist der Patiententisch innerhalb der erwähnten Horizontalebene oder einer Horizontalebene, welche nahe dieser parallel verlaufend ausgebildet ist, frei bewegbar, wie insbesondere um mind. 180° rotierbar um eine Achse, welche in etwa durch das Isozentrum der Protonenstrahlbehandlungsanlage verläuft. Das Isozentrum wird gebildet einerseits durch den von der Protonenstrahlführungs- und -steuerungseinrichtung austretenden Protonenstrahl und andererseits durch die Drehachse, um welche diese Einrichtung rotierbar ist.

Durch diese erfindungsgemässe Anordnung ist einerseits, wie bereits erwähnt, der Patiententisch jederzeit frei zugänglich und zum anderen ist trotzdem eine allseitige Behandlung eines Patienten möglich, indem einerseits eine Behandlung von oben und von unten gewährleistet ist, wie auch die Behandlung von beiden Seiten durch die Rotation des Patiententisches um 180°.

Bevorzugte Ausführungsvarianten der erfindungsgemässen Anordnung sind in den abhängigen Ansprüchen charakterisiert.

Verwendungen der erfindungsgemässen Anordnung sind in den Ansprüchen 11 ff charakterisiert.

Für das bessere Verständnis der Erfindung wird nun eine erfindungsgemässe Protonenstrahl-Behandlungsanlage beispielsweise anhand der Fig. 1 - 3 näher erläutert.

Dabei zeigen:
- Fig. 1: schematisch in Perspektive eine Protonenstrahl-behandlungsanlage bei seitlicher Behandlung eines Patienten,
- Fig. 2: die Anlage aus Fig. 1 bei Behandlung eines Patienten von oben, und
- Fig. 3: die Anlage aus Fig. 1 bei Behandlung eines Patienten von unten.

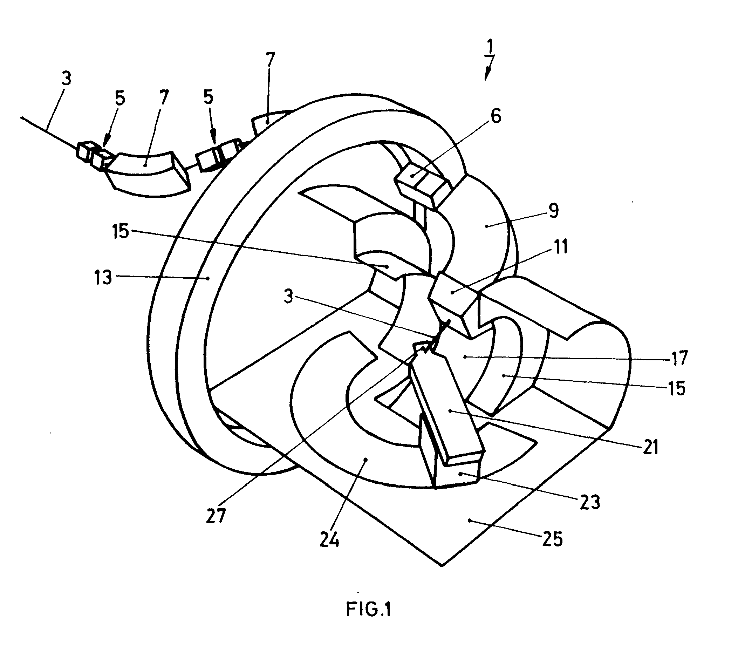

In Fig. 1 ist schematisch und vereinfacht eine Anlage 1 zum Behandeln eines Patienten mittels Protonenstrahltherapie dargestellt. Dabei wird der Protonenstrahl 3 mittels Quadrupolen 5 und Magneten 7 zur eigentlichen endständigen Protonenstrahlführungs- und -steuereinrichtung 9 geführt. Frontseitig an diese Führungs- und Steuereinrichtung 9 angeordnet ist ein Austrittsfenster 11 bzw. eine sogenannte "nozzle", durch welches hindurch der Protonenstrahl auf den Patienten gerichtet austritt. Durch eine zusätzliche Ablenkungsmagnetanordnung 6, auch genannt "sweeper magnet", kann der Protonenstrahl in horizontaler Richtung innerhalb eines engbegrenzten Winkels abgelenkt werden. Am gleichen Ort ist auch ein zweiter "sweeper magnet" inden Zeichnungen dargestellt, welcher als Option für eine zweite schnelle, aber durch den Spalt des 90°-Magneten begrenzte magnetische Bewegung des Strahles wahlweise benützt werden kann. Ebenfalls im Bereich des Austrittsfensters 11 angeordnet und in Fig. 1 nicht erkennbar, ist eine Eindringtiefenverstellvorrichtung oder auch genannt "range shifter", mittels welcher die Eindringtiefe des Protonenstrahls in den Körper eines Patienten eingestellt werden kann. Grundsätzlich sei erneut an dieser Stelle auf die eingangs erwähnte Literatur von Pedroni et al. verwiesen, in welcher die grundsätzliche Funktionsweise einer Protonenstrahltherapieanlage wie der sog. "gantry" am Paul Scherrer Institut beschrieben ist.

Weiter in Fig. 1 erkennbar ist eine Führungsschiene 13, an welcher um eine zentrale Rotationsachse bewegbar die Protonenstrahlführungs- und -steuereinrichtung 9 angeordnet ist. Durch seitliche Abschirmungsführungen 15 hindurch ragend bewegt sich dabei das Austrittsfenster 11 in einer schlitzartige Oeffnung 17 beim Bewegen der Führungs- und Steuerungseinrichtung (9) entlang der Halterung 13.

In einer Horizontalebene liegend in etwa verlaufend durch die Drehachse der Strahlführungs- und -steuerungseinrichtung ist ein Patiententisch 21 angeordnet. Dieser ist um eine Drehachse und auf einer Halterung 23 entlang einer Führung 24 bewegbar, wobei diese Führung auf einer Arbeitsplattform 25 angeordnet ist. Die Rotation des Patiententisches 21 erfolgt dabei vorzugsweise um eine Rotationsachse, welche in etwa durch den kopfseitigen Bereich 27 des Patiententisches 21 verläuft, und welche Rotationsachse weitgehendst durch den Bereich des sog. Isozentrums der Anlage verläuft. Selbstverständlich kann die Horizontalebene, in welcher der Patiententisch 21 angeordnet ist, auch beabstandet parallel oberhalb oder unterhalb der Horizontalebene verlaufen, durch welche die Drehachse der Strahlführungs- und -steuerungseinrichtung 9 verläuft. Jedoch soll dieser Abstand derart begrenzt sein, dass einerseits eine gute Behandlung von oben und von unten möglich ist und zudem der Patiententisch in angemessener Höhe von der Arbeitsplattform 25 aus durch eine Betreuungsperson erreicht werden kann. Selbstverständlich ist es auch möglich, dass der Patiententisch 21 auf der Halterung 23 in der Höhe verstellbar angeordnet ist sowie in Längs- und Querrichtung des Tisches verschiebbar.

Die Drehbarkeit des Patiententisches sollte mindestens einen Winkel von 180° mit einschliessen, allerdings ist aus Fig. 1 deutlich erkennbar, dass ein wesentlich grösserer Winkel als 180° aus konstruktiven Gründen nicht machbar und im übrigen auch nicht notwendig ist. Gemäss einer speziellen Ausführungsvariante ist es zudem auch möglich, den Patiententisch um eine weitere Drehachse rotierbar auszubilden, wie beisielsweise um eine mittig im Tisch verlaufende senkrechte Drehachse. Diese Rotation ist beispielsweise dann notwendig bzw. sinnvoll, wenn ein Patient im Beinbereich zu behandeln ist und somit dieser gegen das Isozentrum der Anlage auszurichten ist, damit mittels Protonenstrahl entsprechend beispielsweise ein Tumor in einem Bein behandelt werden kann.

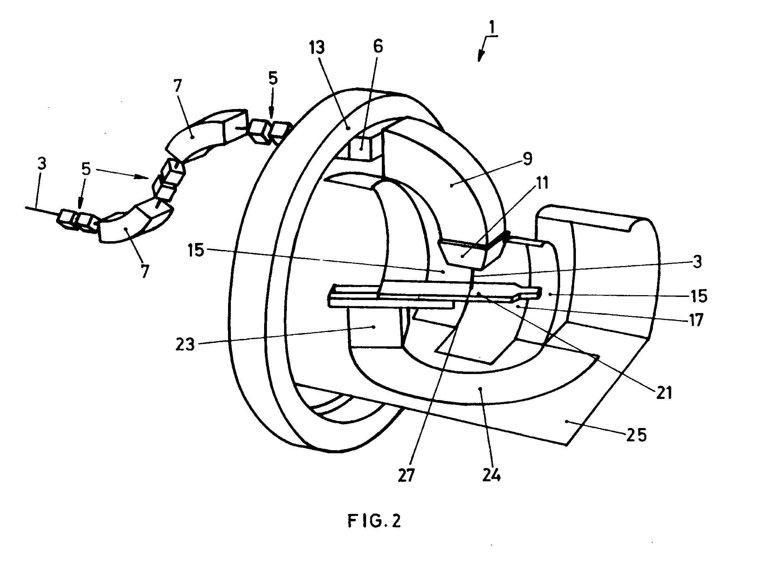

Entsprechend der Anlage in Fig. 1 zeigt Fig. 2 dieselbe Anlage mit der Strahlführungs- und -steuerungseinrichtung 9 von oben gerichtet angeordnet. Mit anderen Worten erfolgt die Protonenstrahlbehandlung gemäss der Anordnung in Fig. 2 von oben, wobei zusätzlich der Patiententisch in einer gegenüber Fig. 1 veränderten Position dargestellt ist. Zudem ist in Fig. 2 deutlich erkennbar, dass der Patiententisch in Längsrichtung des Tisches verschiebbar ist.

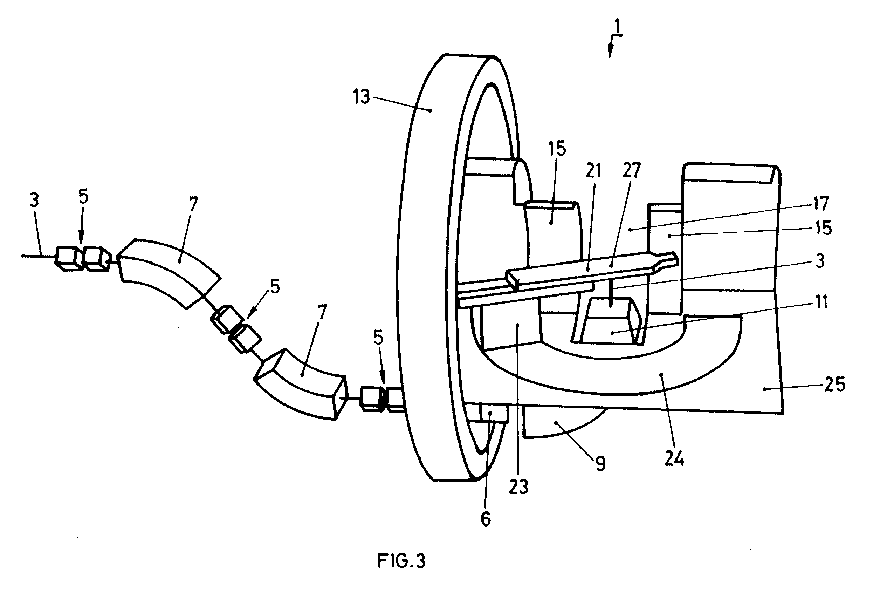

Schliesslich zeigt Fig. 3 erneut eine weitere Positionierung der Strahlführungs- und -steuerungseinrichtung 9, indem eine Behandlung von unterhalb des Patienten zu erfolgen hat.

Im Vergleich mit der bekannten sog. "gantry" Anlage beim Paul Scherrer Institut zeigt sich sofort der wesentliche Vorteil der erfindungsgemäss beschriebenen Anlage, indem der Patiententisch beispielsweise für Behandlungen von unten nicht wesentlich angehoben werden muss und somit jederzeit die Zugänglichkeit von einer Betreuungsperson zum Patiententisch gewährleistet ist. Dies bringt nicht nur Vorteile für einen zu behandelnden Patienten, sondern auch für eine Betreuungsperson, indem in der erfindungsgemäss vorgeschlagenen Anlage keine Unfallgefahr mehr durch Abstürzen in einen Schacht besteht.

Eine weitere Problematik bei bestehenden Protonenbehandlungsanlagen besteht im Bereich des Austrittsfensters des Protonenstrahlgehäuses, im Englischen und im Sprachjargon auch "nozzle" genannt. Im Bereich dieses Austrittsfensters ist bei der eingangs beschriebenen Anlage eine Eindringtiefenverstellvorrichtung oder auch genannt "range shifter" angeordnet, mittels welcher die Eindringtiefe des Protonenstrahles genauestens gesteuert wird, da die für die Zerstörung eines kranken Organes bzw. eines Tumors notwendige Energie exakt am Ende der Reichweite des Protonenstrahles abgegeben wird.

In der Praxis hat es sich nun gezeigt, dass durch den Luftspalt zwischen dem sogenannten "range shifter" und dem Patienten der Protonenstrahl gestört wird, womit die Exaktheit der Strahlenführung zumindest leicht gestört ist.

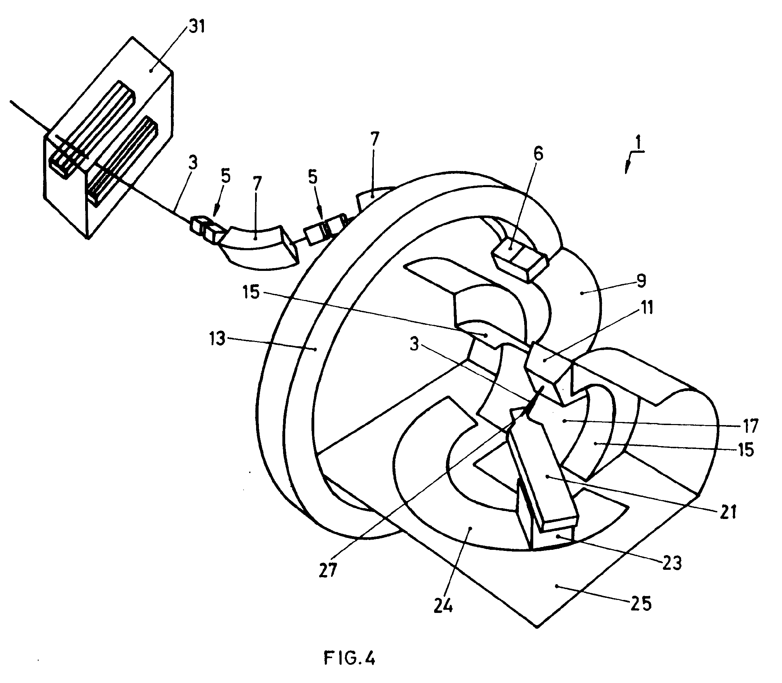

Aus diesem Grunde wird weiter erfindungsgemäss vorgeschlagen, diese Verstelleinrichtung zur Beeinflussung der Reichweite des Protonenstrahles bzw. den sogenannten "range shifter" nicht mehr im Bereich des Ausgabefensters bzw. der sogenannten "nozzle" am Protonenstrahlführungsgehäuse anzuordnen, sondern vorgängig der Eingabe des Protonenstrahles in das Führungsgehäuse, in welchem ja bekanntlich der Protonenstrahl in Richtung zum Patienten und zum zu behandelnden sogenannten "spot" geführt wird. Unter Bezug auf Fig. 1 bedeutet dies, dass der sog. "range shifter" nicht mehr im Bereich des Austrittsfenster 11 angeordnet ist, sondern der Behandlungsanordnung 1 vorgeschaltet, wie in Fig. 4 schematisch dargestellt und mit dem Bezugszeichen 31 versehen.

Allerdings hat das Plazieren des sog. "range shifters" vorgängig der nachfolgenden Protonenstrahlführung in der Behandlungsanordnung zur Folge, dass damit gekoppelt ebenfalls die Magnetanordnungen 7 bzw. die im endständigen Protonenstrahlführungs- und -steuerungseinrichtungsgehäuse 9 angeordnete Magnetanordnung variierbar sein müssen, um eine erhöhte bzw. abgeschwächte Energie des Protonenstrahls derart auszugleichen, dass schlussendlich der Protonenstrahl wiederum an den gewünschten Ort in einem Patienten geführt wird. Doch ist dies mit den heute bekannten Prozess-Steuerungen bzw. den bekannten Computer-Steuerungen kein Problem, und andererseits kann durch vereinfachte Konstruktion des Austrittsfensters die eingangs erwähnte Problematik der Exaktheit der Strahlenführung wesentlich verbessert werden.

Ueblicherweise wird für das notwendige Zerstören der kranken Zellen in einem Organ bzw. in einem menschlichen Körper der Patiententisch in bezug auf das Protonenstrahlführungsgehäuse in diskreten Schritten bewegt, damit mittels des Protonenstrahls punktweise der ganze behandelnde Bereich im Organ bzw. menschlichen Körper bestrichen werden kann. Diese Bewegung des Patiententisches ist deshalb notwendig, da durch "sweeper magnet" und "range shifter" lediglich das Bewegen des Protonenstrahles in zwei Richtungen bzw. zweidimensional erfolgen kann, so dass für das räumliche Behandeln eines Bereiches in einem Patienten bzw. für die dritte Dimension der Patiententisch bewegbar ausgestaltet werden muss. Durch die gewählte spot-scanning-Technik erfolgt diese Bewegung des Patiententisches nicht kontinuierlich, sondern wie erwähnt in diskreten Schritten. Dieser diskrete Bewegungsablauf wird vielfach als nachteilig bzw. störend beurteilt, wie insbesondere von behandelnden Aerzten bzw. von Betreuungspersonen.

Aus diesem Grunde wird gemäss einer weiteren erfindungsgemässen Ausführungsvariante der Protonentherapieanlage vorgeschlagen, im Bereich des Austrittsfensters bzw. der sogenannten "nozzle" ein Abdeckungsgehäuse anzuordnen, innerhalb welchem nicht sichtbar sämtliche für die Dosierung und Steuerung bzw. Abschirmung notwendigen Einrichtungen und Elemente für das Steuern des Protonenstrahls angeordnet sind. Dieses Gehäuse selbst ist bewegungsmässig mit dem Patiententisch über eine Steuerung verbunden, so dass die diskreten Bewegungen des Tisches auch durch dieses Abdeckungsgehäuse ausgeführt werden und für den Patienten eine Relativbewegung in bezug auf das Protonenstrahlführungsgehäuse nicht stattfindet. Ein weiterer Vorteil des Anordnens eines derartigen Abdeckungsgehäuses liegt darin, dass jederzeit die relative Lage einer Berührungssicherung, welche integral verbunden mit dem Gehäuse angeordnet sein kann, einen optimalen Schutz gewährleistet, falls der Patiententisch in bezug auf das Austrittsfenster bzw. der "nozzle" bewegt werden sollte. Somit kann eine derartige Sicherung für das Unterbrechen des Protonenstrahls innerhalb Bruchteilen einer Millisekunde, innerhalb des Gehäuses angeordnet werden.

Der Vorteil des Anordnens eines derartigen Abdeckungsgehäuses liegt auch darin, dass beispielsweise bei anderen bekannten Anlagen, wie beispielsweise solche verwendend die sog. scattering- Technik, für die Bündelung und Focussierung des Protonenstrahls notwendige Kollimatoren und Kompensatoren an einem derartigen Gehäuses angeordnet werden können. Durch die gesteuerte Verbindung der Abdeckung mit dem Patiententisch ist in diesem Fall gewährleistet, dass der Protonenstrahl beim Bewegen des Patiententisches trotzdem immer an die richtige Stelle im Körper des Patienten gerichtet bleibt.

Unter Bezug auf Fig. 1 bedeutet dies, dass das schematisch dargestellte Gehäuse 11 des Austrittsfensters nicht fest mit der Protonenstrahlführungs- und -steuereinrichtung 9 verbunden ist, sondern steuerungsmässig entsprechend den Bewegungen des Patiententisches ebenfalls bewegbar ist. Ueber eine Steuerung ist es dabei möglich, die Bewegungen des Abdeckgehäuses 11 mit denjenigen des Patiententisches 21 zu koppeln, derart, dass keine Relativbewegungen zwischen Gehäuse und Tisch stattfinden beim Bewegen des Patiententisches 21 während der Behandlung eines Patienten.

Durch die erfindungsgemäss vorgeschlagenen Verbesserungen an einer Protonenstrahlbehandlungsanlage, wie insbesondere an einer mittels sog. spot-scanning Technik arbeitenden Anlage, wie die sog. "gantry" am Paul Scherrer Institut, ergeben sich wesentliche Vereinfachungen beim Betreiben der Anlage sowie Erhöhung der Sicherheit und Bedienungsfreundlichkeit der Anlage sowohl für Patienten wie auch für das Bedienungspersonal.

## Patentansprüche

1. Anordnung zum Behandeln eines Patienten mittels Protonentherapie, umfassend eine Protonenstrahlführung mittels Magneten (7), Quadrupolen (5) sowie einer endständigen Protonenstrahlführungs- und Steuerungseinrichtung (9) mit einem Austrittsfenster (11), um den Protonenstrahl (3) an die zu behandelnde Stelle im Patienten zu führen bzw. zu richten, sowie einen steuerbar bewegbaren Patiententisch (21), um den Patienten in gewünschter Position in Bezug auf den Protonenstrahl zu bewegen, wobei durch die Lagerung der Protonenstrahlführungs- und Steuerungseinrichtung (9) eine Horizontaldrehachse definiert wird und das Isozentrum der Anordnung im wesentlichen einem Schnittpunkt des Protonenstrahls (3) mit der Horizontaldrehache entspricht,
**dadurch gekennzeichnet, dass**
a) der Patiententisch (21) in einer im wesentlichen horizontal verlaufenden Ebene angeordnet ist und in der im wesentlichen horizontal verlaufenden Ebene um mindestens 180° zumindest annähernd im Isozentrum der Anordnung drehbar gelagert ist,
b) die Protonenstrahlführungs- und Steuerungseinrichtung (9) aus der im wesentlichen horizontal verlaufenden Ebene um mindestens -135° bis mindestens +135°auf der einen Seite des Patiententisches (21) um die Horizontaldrehachse drehbar angeordnet ist, so dass der Patiententisch (21) von der anderen Seite her jederzeit zugänglich bleibt,
und
c) auf der anderen Seite des Patiententisches (21) im nicht von der Schwenkbewegung überstrichenen Bereich eine Arbeitsplattform (25) für den permanenten Zugang zum Patientientisch (21) eingerichtet ist.

2. Anordnung, insbesondere nach Anspruch 1, **dadurch gekennzeichnet, dass** der Patiententisch (21) in demjenigen Bereich der Horizontalebene rotierbar bzw. bewegbar angeordnet ist, durch welchen die Strahlführungs- und -steuerungseinrichtung (9) nicht bewegbar ist bzw. welcher demjenigen anderen Bereich gegenüber liegt, durch welchen hindurch die Strahlführungs- und -steuerungseinrichtung (9) bewegbar ist.

3. Anordnung, insbesondere nach einem der Ansprüche 1 oder 2, **dadurch gekennzeichnet, dass** der Patiententisch vorzugsweise um eine in einem endständigen Bereich am Patiententisch (21) verlaufende Achse (27) rotierbar ist.

4. Anordnung, insbesondere nach einem der Ansprüche 1 - 3, **dadurch gekennzeichnet, dass** der Patiententisch in seiner Längsachse verschieblich bzw. bewegbar angeordnet ist.

5. Anordnung, insbesondere nach einem der Ansprüche 1 - 4, **dadurch gekennzeichnet, dass** der Patiententisch zusätzlich um eine in etwa in einem mittigen Bereich des Tisches verlaufende senkrechte Achse rotierbar ist, quer zur Längsachse bewegbar ist sowie höhenverstellbar ausgebildet ist.

6. Anordnung nach einem der Ansprüche 1 bis 5, **dadurch gekennzeichnet, dass** vorgängig der Anordnung, vorgeschaltet den Magneten (7) und Quadrupolen (5) eine Protonenstrahleindringtiefenverstell-Vorrichtung (31) angeordnet ist, aufweisend ein System von im Protonenstrahl bzw. durch den Protonenstrahl bewegbaren Platten bzw. Lamellen, um die Energie und damit verbunden die Eindringtiefe des Protonenstrahles im Patienten zu steuern bzw. zu begrenzen.

## Claims

1. Apparatus for treating a patient using proton therapy, comprising a proton beam guiding device employing magnets (7), quadrupoles (5), and an end-mounted proton beam guiding and control device (9) with an exit window (11), for guiding or directing the proton beam (3) to the treatment spot in the patient, and a controllably movable patient table (21) for moving the patient to the desired position relative to the proton beam, wherein a horizontal axis of rotation is defined by the positioning of the proton beam guiding and control device (9) and the isocentre of the apparatus corresponds essentially to an intersection of the proton beam (3) with the horizontal axis of rotation, **characterised in that**
a) the patient table (21) is arranged in a plane running essentially horizontally and is mounted in such a way that it is rotatable by an angle of at least 180° in the plane running essentially horizontally at least approximately in the isocentre of the apparatus.
b) the proton beam guiding and control device (9) is arranged to be rotatable about the horizontal axis of rotation by an angle of at least -135° to at least +135° from the plane running essentially horizontally on one side of the patient table (21), so that the patient table (21) remains accessible at all times from the other side,
c) on the other side of the patient table (21) in the region which is not covered by the swivel movement, a work platform (25) is set up for permanent access to the patient table (21).

2. Apparatus according to claim 1, **characterised in that** the patient table (21) is arranged to be rotatable or movable in the region of the horizontal plane through which the beam guiding and control device (9) is not movable, or which region lies opposite the particular other region through which the beam guiding and control device (9) is movable.

3. Apparatus in particular according to one of claims 1 or 2, **characterised in that** the patient table is preferably rotatable about an axis (27) in an end-mounted region on the patient table (21).

4. Apparatus in particular according to one of claims 1 to 3, **characterised in that** the patient table is arranged to be slidable or movable in its longitudinal axis.

5. Apparatus in particular according to one of claims 1 to 4, **characterised in that** the patient table is designed to be additionally rotatable about an axis running vertically in essentially the centre region of the table, to be movable in a direction transverse to the longitudinal axis, and also to be adjustable in height.

6. Apparatus according to one of claims 1 to 5, **characterised in that** a proton beam penetration depth adjustment device (31) is located in front of the apparatus before the magnets (7) and quadrupoles (5), having a system of plates or blades movable in or through the proton beam so as to control or restrict the energy and the associated penetration depth of the proton beam in the patient.

## Revendications

1. Dispositif de traitement d'un patient au moyen d'une thérapie protonique, comprenant un guidage de faisceau protonique au moyen d'aimants ( 7 ), de quadrupôles ( 5 ) ainsi que d'un dispositif ( 9 ) final de guidage et de commande du faisceau protonique ayant un hublot ( 11 ) de sortie pour guider ou diriger le faisceau ( 3 ) protonique sur l'endroit à traiter du patient, ainsi qu'une table ( 21 ) pour patient mobile et pouvant être commandée, pour mettre le patient dans la position souhaitée par rapport au faisceau protonique, dans lequel il est défini, par le montage du dispositif ( 9 ) de guidage et de commande du faisceau protonique, un axe de rotation horizontal et l'isocentre du dispositif correspond sensiblement à un point d'intersection du faisceau ( 3 ) protonique avec l'axe de rotation horizontal, **caractérisé en ce que**
a) la table ( 21 ) pour le patient est disposée dans un plan s'étendant sensiblement horizontalement et est montée tournante, dans le plan s'étendant sensiblement horizontalement, d'au moins 180° au moins à peu près à l'isocentre du dispositif,
b) le dispositif ( 9 ) de guidage et de commande du faisceau protonique est monté tournant autour de l'axe de rotation horizontal, hors du plan s'étendant sensiblement horizontalement, d'au moins -135° jusqu'à au moins +135° sur l'un des côtés de la table ( 1 ) pour le patient, de manière à ce que la table ( 21 ) pour le patient reste accessible à tout instant à partir de l'autre côté, et
c) sur l'autre côté de la table ( 21 ) pour le patient est disposée, dans la zone qui n'est pas balayée par le mouvement de pivotement, une plateforme ( 25 ) de travail pour l'accès permanent à la table ( 21 ) pour le patient.

2. Dispositif, notamment suivant la revendication 1, **caractérisé en ce que** la table ( 21 ) pour le patient est montée tournante ou mobile dans la zone du plan horizontal, dans laquelle le dispositif ( 9 ) de guidage et de commande du faisceau n'est pas mobile ou qui est opposée à l'autre zone, dans laquelle le dispositif ( 9 ) de guidage et de commande du faisceau est mobile.

3. Dispositif, notamment suivant l'une des revendications 1 ou 2, **caractérisé en ce que** la table pour le patient peut tourner, de préférence autour d'un axe ( 27 ) vertical s'étendant dans une zone d'extrémité de la table ( 21 ) pour le patient.

4. Dispositif, notamment suivant l'une des revendications 1 à 3, **caractérisé en ce que** la table pour le patient est montée coulissante ou mobile suivant son axe longitudinal.

5. Dispositif, notamment suivant l'une des revendications 1 à 4, **caractérisé en ce que** la table pour le patient peut, en outre, tourner autour d'un axe vertical s'étendant à peu près dans une zone du milieu de la table, être mobile transversalement à l'axe longitudinal, ainsi qu'être réglable en hauteur.

6. Dispositif suivant l'une des revendications 1 à 5, **caractérisé en ce qu'**en avant du dispositif, en amont des aimants ( 7 ) et des quadrupôles ( 5 ), est monté un dispositif ( 31 ) de réglage de la profondeur de pénétration du faisceau protonique ayant un système de plaques et lames mobiles dans le faisceau protonique ou à travers le faisceau protonique, pour commander ou limiter l'énergie et ainsi la profondeur de pénétration du faisceau protonique dans le patient que cela emporte.
